# EUROPEAN PATENT SPECIFICATION

(11) **EP 3 904 534 B1**
(45) Date of publication and mention of the grant of the patent: **10.12.2025**
(21) Application number: 19902039.7
(22) Date of filing: 27.12.2019
(51) Int. Cl.: C12Q 1/6883, G01N 33/68, C12Q 1/6886, C12Q 1/689, G01N 33/74

(54) **GENETIC MARKER FOR HELICOBACTER PYLORI-ASSOCIATED DISEASE**
GENETISCHER MARKER FÜR HELICOBACTER PYLORI-ASSOZIIERTE KRANKHEIT
MARQUEUR GÉNÉTIQUE DE MALADIE ASSOCIÉE À HELICOBACTER PYLORI

(30) Priority: 28.12.2018 KR 20180171846
(43) Date of publication of application: 03.11.2021
(73) Proprietor: CJ Cheiljedang Corporation, Seoul 04560 (KR)
(72) Inventor: LEE, Dong Yun, Hwaseong-si, Gyeonggi-do 18503 (KR); CHOI, Seung Hye, Yongin-si, Gyeonggi-do 16833 (KR); OH, Ji Young, Yongin-si, Gyeonggi-do 16949 (KR)
(74) Representative: Ipsilon
(86) International application number: PCT/KR2019/018607
(87) International publication number: WO 2020/139021

(56) References cited:
- WO-A1-2018/087419
- KR-B1- 101 108 282
- US-A1- 2014 206 023
- US-B2- 8 785 402
- "AFFYMETRIX GENECHIP HUMAN GENOME U.133", 11 March 2002 (2002-03-11), XP008136197, Retrieved from the Internet <URL:http://www.ncbi.nlm.nih.gov/geo/query/acc.cgi?acc=GPL96>
- CHIARA DEGIROLAMO ET AL: "Therapeutic potential of the endocrine fibroblast growth factors FGF19, FGF21 and FGF23", NATURE REVIEWS DRUG DISCOVERY, vol. 15, no. 1, 31 January 2016 (2016-01-31), GB, pages 51 - 69, XP055722052, ISSN: 1474-1776, DOI: 10.1038/nrd.2015.9
- MIGYEONG JEONG ET AL: "Dietary prevention of <i>Helicobacter pylori</i>-associated gastric cancer with kimchi", ONCOTARGET, vol. 6, no. 30, 10 August 2015 (2015-08-10), pages 29513 - 29526, XP055596252, DOI: 10.18632/oncotarget.4897
- PARK JONG MIN ET AL: "Transcriptome profiling implicated in beneficiary actions of kimchi extracts against <i>Helicobacter pylori</i> infection", JOURNAL OF CLINICAL BIOCHEMISTRY AND NUTRITION., vol. 69, no. 2, 1 January 2021 (2021-01-01), JP, pages 171 - 187, XP055950270, ISSN: 0912-0009, DOI: 10.3164/jcbn.20-116
- S MAEDA, K OGURA, H YOSHIDA, F KANAI, T IKENOUE, N KATO, Y SHIRATORI, M OMATA: "Major virulence factors, VacA and CagA, are commonly positive in Helicobacter pylori isolates in Japan", GUT, vol. 42, no. 3, 1 January 1998 (1998-01-01), pages 338 - 343, XP055722049, ISSN: 1468-3288, DOI: 10.1136/gut.42.3.338
- CHIARA DEGIROLAMO, CARLO SABBÀ , ANTONIO MOSCHETTA: "Therapeutic potential of the endocrine fibroblast growth factors FGF19, FGF2I and FGF23", NATURE REVIEWS DRUG DISCOVERY, vol. 15, no. 1, 31 January 2016 (2016-01-31), pages 51 - 69, XP055722052, ISSN: 1474-1776, DOI: 10.1038/nrd.2015.9
- STEPHANE BENOIT , ROBERT J. MAIER: "Dependence of Helicobacter pylori Urease Activity on th e Nickel-Sequestering Ability of the UreE Accessory Protein", JOURNAL OF BACTERIOLOGY, vol. 185, no. 16, 1 August 2003 (2003-08-01), pages 4787 - 4795, XP055722057, ISSN: 0021-9193, DOI: 10.1128/JB.185.16.4787-4795.2003

## Description

### Technical Field

The present disclosure relates to a genetic marker which can be effectively used for the diagnosis of a *Helicobacter* pylori-associated disease.

### BACKGROUND ART

In 1983, Australian microbiologists Marchall and Warren first discovered a curved bacterium in gastric mucosal biopsy tissues in patients with type B gastritis, and then cultivated the bacterium and identified that it was a new species associated with *Campylobacter* genus. Since then, active research has been conducted on the association between *Helicobacter pylori* and upper gastrointestinal diseases such as gastritis, peptic ulcer, and the like. *Helicobacter pylori* is a Gram-negative bacillus, has flagella surrounded by a thin membrane, releases a large amount of urease, and is known to be spread through a fecal-oral route, and is a pathogen that has attracted great interest worldwide because the World Health Organization (WHO) identified it as a definite carcinogen in 1994.

Currently, the pathogenesis of a disease caused by *Helicobacter pylori* is known as follows: bacteria that entered the gastric lumen along with food penetrate the gastric mucus layer by flagella having strong motility, and inhabit the upper epithelial layer and the junction between cells in the gastric mucus layer, and release a large amounts of urease to change the mucus layer to alkaline, thereby leading to abnormally excessive stimulation of gastric acid secretion by gastrin, and in turn, resulting in inflammation and an ulcer. In addition, according to a recent study that found that *Helicobacter pylori* is an important risk factor for the pathogenesis of gastric adenocarcinoma, the World Health Organization has designated *Helicobacter pylori* as a group I carcinogen.

In particular, infection with *Helicobacter pylori* has been reported to occur frequently in developing countries including South Korea. According to the national epidemiological survey conducted on 5,732 people with no upper gastrointestinal tract symptoms, the overall prevalence of *Helicobacter pylori* infection was 46.6%, and the infection rate was 69.4% in adults over 16 years old and was especially high when people were aged in their 40s (78.5%). In addition, according to the investigation conducted on 1,031 patients with peptic ulcer, 66% of gastric ulcer patients and 79% of duodenal ulcer patients were found to be infected with *Helicobacter pylori* and 71% of patients with gastric and duodenal ulcers were reported to be infected with *Helicobacter pylori.*

Many disease states are characterized by differences in the expression levels of various genes either through changes in the copy number of the genetic DNA or through changes in levels of transcription of particular genes (e.g., through control of initiation, provision of RNA precursors, RNA processing, etc.). For example, losses and gains of genetic material play an important role in malignant transformation and progression. These gains and losses are thought to be "driven" by at least two kinds of genes, oncogenes and tumor suppressor genes. Oncogenes are positive regulators of tumorigenesis, while tumor suppressor genes are negative regulators of tumorigenesis. Thus, changes in the expression (transcription) levels of particular genes (e.g., oncogenes or tumor suppressor genes) serve as signposts for the presence and progression of various cancers.

The *Helicobacter pylori* infection reverses some, or all, of these gene expression patterns. The expression pattern change of some or all of these genes may therefore, be used as a method to monitor or predict the efficacy of medicines. The analysis of the gene expression changes may be performed in the target tissue of interest (e.g., tumor) or in some surrogate cell population (e.g., peripheral blood leukocytes). In the latter case, correlation of the gene expression changes with efficacy (e.g., tumor shrinkage or non-growth) must be especially strong for the gene expression change pattern to be used as a marker for efficacy. Many conventional diagnostic preparations for detecting *Helicobacter pylori* infection are known in the art (Patent Document 1 and Patent Document 2).

The Affymetrix family of GeneChip Human Genome U.133 Arrays allows to analyze gene expression across the whole genome(https://www.ncbi.nlm.nih.gov/geo/query/acc.cgi?acc=GPL 96).

Chiara Degirolamo *et al.* disclose several studies in which the level of FGF-21 gene or corresponding protein level have been determined ("Therapeutic potential of the endocrine fibroblast growth factors FGF19, FGF21 and FGF23", NATURE REVIEWS DRUG DISCOVERY, 2016, 15(1) : 51-69).

Document US 2014/206023 discloses an antibody for detecting FGF-21 in a sample.

Migyeong Jeong *et al.* disclose the study of *H. pylory* induced gastric cancer and the effect of treatment using kimchi extracts ("Dietary prevention of iHelicobacter pylori/i-associated gastric cancer with kimchi", ONCOTARGET, 2015, 6(30): 29513-26).

To date, however, information on a group of specific genes whose expression patterns may be changed due to *Helicobacter pylori* infection, and a method for diagnosing or predicting the development, progression, and prognosis of a Helicobacter-pylori-associated disease by using the information have been hardly known.

### Prior art documents

### Patent Documents

(Patent Document 1) Korean Unexamined Publication No. 2003-0001506
(Patent Document 2) Korean Unexamined Publication No. 2000-0033013

### DISCLOSURE OF THE INVENTION

### TECHNICAL PROBLEM

An object of the present disclosure is to provide: a composition for diagnosing a *Helicobacter* pylori-associated disease, the composition comprising a preparation for measuring the expression amount of a gene whose expression is increased or decreased by *Helicobacter pylori* infection; and a method for assisting the diagnosis, prediction of prognosis, and determination of treatment strategies in a patient having a *Helicobacter* pylori-associated disease by using the composition.

In addition, another object of the present disclosure is to provide: a method for identifying and analyzing a group of genes whose expressions are specifically changed by *Helicobacter pylori* infection in a mammalian tissue or cell sample; and a method for diagnosing a *Helicobacter pylori-*associated disease and predicting the treatment effect in a mammalian subject by using the group of genes as a biomarker.

### TECHNICAL SOLUTION

In order to achieve the above object, one aspect of the present disclosure provides a composition and a kit for diagnosing a *Helicobacter* pylori-associated disease, comprising a preparation for measuring the expression amount of FGF21 (fibroblast growth factor 21, Gene ID: 26291) gene, whose expression is increased or decreased by *Helicobacter pylori* infection.

Another aspect of the present disclosure provides a method for providing information on the diagnosis or prognosis prediction, comprising the step of measuring the amount of mRNA of a gene whose expression is increased or decreased by *Helicobacter pylori* infection or a protein expressed from the gene, present in a biological sample of a patient infected with *Helicobacter pylori* and comparing the same with the amount measured in a sample of a normal subject.

The present invention is set out in the appended set of claims.

### ADVANTAGEOUS EFFECTS

In the present disclosure, a group of genes whose expressions are specifically increased or decreased in relation to *Helicobacter pylori* infection was identified and when *Helicobacter pylori* infection was overcome, it was found that the expressions of the group of genes were restored to normal states. Thus, the composition and the kit of the present invention as defined in claims 1 to 4, can be effectively used for the diagnosis or prognosis prediction of a *Helicobacter* pylori-associated disease.

### BRIEF DESCRIPTION OF THE DRAWINGS

FIG. 1 is a graph showing the types of genes whose expressions are specifically increased by *Helicobacter pylori* infection.
FIG. 2 is a heatmap and electrophoresis picture showing a list of genes whose expressions were restored to normal states when a cell line, which was infected with *Helicobacter pylori* and thus in which the expressions of specific genes were increased, was treated with the kimchi extract of the present disclosure.
FIG. 3 is a graph showing the types of genes whose expressions are specifically decreased by *Helicobacter pylori* infection.
FIG. 4 is a heatmap and electrophoresis picture showing a list of genes whose expressions were restored to normal states when a cell line, which was infected with *Helicobacter pylori* and thus in which the expressions of specific genes were decreased, was treated with the kimchi extract of the present disclosure.
FIG. 5 is a graph showing that the increased expressions of genes for ER stress due to *Helicobacter pylori* infection were restored to normal states by treatment of the kimchi extract of the present disclosure in an animal model.
FIG. 6 is a graph showing that the decreased expressions of genes for antioxidation due to *Helicobacter pylori* infection were restored to normal states by treatment of the kimchi extract of the present disclosure in an animal model.

### MODE FOR CARRYING OUT THE INVENTION

Hereinafter, the present application will be specifically described.

Unless defined otherwise herein, all technical and scientific terms used herein have the same meaning as commonly understood by a person skilled in the art to which this invention belongs. Generally, the nomenclature used herein and the experimental methods which will be described below are those well-known and commonly employed in the art.

Hereinafter, the present invention will be described in more detail.

### 1. A composition and a kit for diagnosing a Helicobacter pylori-associated disease

The present disclosure provides: a composition for diagnosing a *Helicobacter* pylori-associated disease, the composition comprising a preparation for measuring the expression amount of a gene whose expression is increased or decreased by *Helicobacter pylori* infection; and a kit for diagnosing a *Helicobacter* pylori-associated disease, comprising the composition. In one embodiment of the present disclosure, the gene whose expression is increased by *Helicobacter pylori* infection may be selected from the group consisting of FGF21(fibroblast growth factor 21, Gene ID: 26291), CTH (cystathionine g-lyase, Gene ID: 1491), CREBRF (cAMP responsive element binding protein, Gene ID: 153222), DLL4 (delta-like 4, Gene ID: 54567), FGF18 (fibroblast growth factor 18, Gene ID: 8817), FOS (Fos proto-oncogene, AP-1 transcription factor subunit, Gene ID: 2353), PDK1 (phosphoinositide-dependent kinase-1, Gene ID: 5170), PTPRN (receptor-type tyrosine-protein phosphatase-like N, Gene ID: 5798), CLIC4 (chloride intracellular channel 4, Gene ID: 25932), PTPRB (protein tyrosine phosphatase, receptor type B, Gene ID: 5787), PPP1R15A (protein phosphatase 1 regulatory subunit 15A, Gene ID: 23645), PTPRH (protein tyrosine phosphatase, receptor type H, Gene ID: 5794), DDIT3 (DNA damage inducible transcript 3, Gene ID: 1649), BIRC3 (baculoviral IAP repeat containing 3, Gene ID: 330), FOXO3 (forkhead box O3, Gene ID: 2309), BCL2 (BCL2, apoptosis regulator, Gene ID: 596), PRKCE (protein kinase C epsilon, Gene ID: 5581), CHMP4C (charged multivesicular body protein 4C, Gene ID: 92421), CLDN14 (claudin 14, Gene ID: 23562), SLC7A11 (solute carrier family 7 member 11, Gene ID: 23657), BOC (BOC cell adhesion associated, oncogene regulated, Gene ID: 91653), AJUBA (ajuba LIM protein, Gene ID: 84962), LMO7 (LIM domain 7, Gene ID: 4008), MMP24 (matrix metallopeptidase 24, Gene ID: 10893), C3orf58 (divergent protein kinase domain 2A, Gene ID: 205428), KLF10 (Kruppel like factor 10, Gene ID: 7071), CSGALNACT1 (chondroitin sulfate N-acetylgalactosaminyltransferase 1, Gene ID: 55790), CEP120 (centrosomal protein 120, Gene ID: 153241), CHAC1 (ChaC glutathione specific gamma-glutamylcyclotransferase 1, Gene ID: 79094), ASNS (asparagine synthetase (glutamine-hydrolyzing), Gene ID: 440), NFE2L2 (nuclear factor, erythroid 2 like 2, Gene ID: 4780), RIOK3 (RIO kinase 3, Gene ID: 8780), TXNIP (thioredoxin interacting protein, Gene ID: 10628), MTR (5-methyltetrahydrofolate-homocysteine methyltransferase, Gene ID: 4548), IFRD1 (interferon related developmental regulator 1, Gene ID: 3475), and a combination thereof, but the present disclosure is not limited thereto. Determining whether the expression of the gene is increased or not allows the diagnosis of *Helicobacter pylori* infection. In one embodiment of the present disclosure, the gene whose expression is increased by *Helicobacter pylori* infection may be selected from the group consisting of FGF21, CTH, CREBRF, PTPRN and a combination thereof, but the present application is not limited thereto. In one embodiment of the present disclosure, the gene whose expression is increased by *Helicobacter pylori* infection is FGF21.

In another embodiment of the present disclosure, the gene whose expression is decreased by *Helicobacter pylori* infection may be selected from the group consisting of ADGRA2 (adhesion G protein-coupled receptor A2, Gene ID: 25960), TBX4 (T-box 4, Gene ID: 9496), OVOL2 (ovo like zinc finger 2, Gene ID: 58495), ACVRL1 (activin A receptor like type 1, Gene ID: 94), ALB (albumin, Gene ID: 213), JADE1 (jade family PHD finger 1, Gene ID: 79960), MLLT11 (MLLT11, transcription factor 7 cofactor, Gene ID: 10962), ADORA1 (adenosine A1 receptor, Gene ID: 134), TNFRSF8 (TNF receptor superfamily member 8, Gene ID: 943), NLRP12 (NLR family pyrin domain containing 12, Gene ID: 91662), CASP14 (caspase 14, Gene ID: 23581), LTA (lymphotoxin alpha, Gene ID: 4049), SMAGP (small cell adhesion glycoprotein, Gene ID: 57228), NEO1 (neogenin precursor1, Gene ID: 4756), MTSS1 (MTSS1, I-BAR domain containing, Gene ID: 9788), TSPAN32 (tetraspanin 32, Gene ID: 10077), CLDN8 (claudin 8, Gene ID: 9073), MYBPH (myosin binding protein H, Gene ID: 4608), SGCE (sarcoglycan epsilon, Gene ID: 8910), CDH15 (cadherin 15, Gene ID: 1013), ARHGAP6 (Rho GTPase activating protein 6, Gene ID: 395), ZFP36L2 (ZFP36 ring finger protein like 2, Gene ID: 678), EHF (ETS homologous factor, Gene ID: 26298), SLAMF6 (SLAM family member 6, Gene ID: 114836), HLA-DPB1 (major histocompatibility complex, class II, DP beta 1, Gene ID: 3115), SSC5D (scavenger receptor cysteine rich family member with 5 domains, Gene ID: 284297), CCR4 (C-C motif chemokine receptor 4, Gene ID: 1233), CCR7 (C-C motif chemokine receptor 7, Gene ID: 1236), KLRG1 (killer cell lectin-like receptor subfamily G member 1, Gene ID: 10219), BLNK (B cell linker, Gene ID: 29760), IGFBP1 (insulin-like growth factor=binding protein 1, Gene ID: 3484), GIF (MIF, macrophage migration inhibitory factor, Gene ID: 4282), and a combination thereof, but the present disclosure is not limited thereto. Determining whether the expression of the gene is decreased or not allows the diagnosis of *Helicobacter pylori* infection.

In the present disclosure, the nucleotide sequence of the gene whose expression is increased or decreased by *Helicobacter pylori* infection as exemplified above, and the amino acid sequence of a protein expressed from the gene can be easily obtained by a person skilled in the art from published database such as the GenBank of NCBI or documents. Specifically, Gene ID of the present application can be identified by using unique identifiers (UID) in the National Center for Biotechnology Information (NCBI) via NCBI gene search (https://www.ncbi.nlm.nih.gov/gene/).

In one embodiment of the present disclosure, the preparation for measuring the expression amount of the gene whose expression is increased or decreased by *Helicobacter pylori* infection may be a preparation for measuring the amount of mRNA of the gene or the amount of the protein expressed from the gene. The preparation for measuring the amount of mRNA of the gene refers to a preparation capable of: specifically binding to and recognizing mRNA of the gene; or amplifying the amount of mRNA of the gene. As a specific example, it may be, but is not limited to, a pair of probes or a probe specifically binding to the nucleotide sequence of mRNA or cDNA prepared by reverse transcription of the mRNA.

In the present disclosure, the "primer" refers to a short nucleic acid sequence having a free 3'-end hydroxyl group, with which a complementary template strand forms a base pair and thus which serves to provide a starting point when a nucleic acid polymerase replicates and amplifies the template strand. The "probe" refers to a nucleic acid fragment that is several to hundreds of bases in length, consisting of a sequence capable of specifically binding to mRNA or cDNA.

In one embodiment of the present disclosure, the amount of mRNA of the gene may be measured by methods such as PCR, RT-PCR, competitive RT-PCR, and real-time RT-PCR using sense and antisense primers of the gene sequence, and may be measured by methods such as Northern blotting and microarray using a probe having a sequence capable of specifically binding to mRNA of the gene or cDNA prepared by reverse transcription, and furthermore, may be measured by methods such as RNase protection assay and sequencing, but the present application is not limited thereto, and any preparation required for using any methods known to a person skilled in the art may be used.

The preparation for measuring the amount of protein expressed from the gene refers to a preparation capable of specifically binding to and recognizing the protein. As a specific example, it may be, but is not limited to, an antibody or aptamer specifically binding to the protein.

The "antibody" refers to an immunoglobulin molecule that immunologically specifically binds to an epitope of the protein and has reactivity, and it is meant to include without limitation, a monoclonal antibody, a polyclonal antibody, an antibody with a full-length chain structure, an antibody of a functional fragment having at least antigen-binding function, and a recombinant antibody. The aptamer refers to a single-stranded nucleic acid molecule having a stable three dimensional structure with a characteristic capable of targeting and specifically binding to the protein, and the aptamer specific for the protein may be synthesized by using systemic evolution of ligands by exponential enrichment (SELEX) technology, and the like.

In one embodiment of the present disclosure, the amount of the protein expressed from the gene may be measured by methods such as Western blotting, protein microarray (protein chip), enzyme linked immunosorbent assay (ELISA), 2-dimensional electrophoresis, immunohistochemistry (IHC), immunofluorescence, co-immunoprecipitation assay, fluorescence activated cell sorter (FACS), radioimmunoassay (RIA), radioimmunodiffusion, matrix assisted laser desorption/ionization time of flight mass spectrometry (MALDI-TOF), and the like, using the antibody or aptamer, but the present disclosure is not limited thereto, and any preparation required for using any methods known to a person skilled in the art may be used.

The composition comprising the preparation for measuring the amount of the gene expression can be used for the diagnosis of a *Helicobacter* pylori-associated disease, as well as for the prediction of the prognosis of a patient having the disease. In one embodiment of the present disclosure, when the amount of the gene expression is measured and as a consequence, the amount of the gene expression is increased or decreased, it is possible to predict that the patient having the *Helicobacter* pylori-associated disease will have poor prognosis. Thus, establishment of strategies and countermeasures for the treatment is possible.

In the present disclosure, the term "diagnosis" is to identify the presence or characteristic of a pathological condition, and for the purpose of the present disclosure, it refers to determination of a difference in therapeutic effect against cancer depending on metastasis of a cancer patient, as well as, determination whether the corresponding subject has recurrence, drug response, resistance, etc. after cancer treatment. Preferably, when the mutations of the genes of the present disclosure are used, it is also possible to predict a difference in survival rate by checking whether there are mutations in a specimen of a patient having kidney cancer. In this case, a difference in therapeutic effect against kidney cancer depending on metastasis of the corresponding patient having kidney cancer and the prognosis of the corresponding patient in the future may be determined from the difference in survival rate.

In the present disclosure, the term "prognosis" refers to the progress and full recovery of a disease, including, for example, the relapse and metastatic spread of a *Helicobacter* pylori-associated disease, and drug resistance. In one embodiment of the present disclosure, the *Helicobacter pylori-*associated disease may include, but is not limited to, gastritis, gastric ulcer, gastric cancer, and the like.

In the present disclosure, the term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers.

In the present disclosure, the term "gene" and modified products thereof include DNA fragments involved in the synthesis of polypeptide chains, and includes regions upstream and downstream from a coding region, for example, a promoter and a 3'-untranslated region, respectively, and also includes intervening sequences (introns) between respective coding fragments (exons).

In one embodiment of the present disclosure, the mutation of the gene may include any one or more mutations, and may, for example, have at least one mutation selected from the group consisting of truncating mutation, missense mutation, nonsense mutation, frameshift mutation, in-frame mutation, splice mutation, and splice region mutation. The frameshift mutation may be at least one selected from a frameshift insertion (FS ins) mutation and a frameshift deletion (FS del) mutation. The in-frame mutation may be at least one selected from an in-frame insertion (IF ins) mutation and an in-frame deletion (IF del) mutation.

The terminology "X#Y" in the context of a mutation in a polypeptide sequence is obviously recognized by a person skilled in the art, where "#" indicates the location of the mutation in terms of the amino acid number of the polypeptide, "X" indicates the amino acid found at that position in the wild-type amino acid sequence, and "Y" indicates the mutant amino acid at that position.

Examples of an analytical method for diagnosing the prognosis of a *Helicobacter* pylori-associated disease by using the increase or decrease in the gene expression, are a next-generation sequencing (NGS) method, RT-PCR, a direct nucleic acid sequencing method, a microarray, and the like, but the present disclosure is not limited thereto. In one embodiment of the present disclosure, the antibody or nucleic acid probe for determining the increase or decrease in the gene expression is detectably labeled. The label may be selected from the group consisting of an immunofluorescent label, a chemiluminescent label, a phosphorescent label, an enzyme label, a radioactive label, avidin/biotin, colloidal gold particles, colored particles and magnetic particles, but the present application is not limited thereto. In another embodiment of the present disclosure, the increase or decrease in the gene expression may be determined by means of radioimmunoassay, Western blot assay, an immunofluorescence assay, an enzyme immunoassay, an immunoprecipitation assay, a chemiluminescence assay, an immunohistochemical assay, a dot-blot assay, a slot-blot assay, or a flow cytometric assay, but the present application is not limited thereto.

In the present disclosure, the term "polynucleotide" generally refers to any polyribonucleotide or polydeoxyribonucleotide that may be unmodified RNA or DNA or modified RNA or DNA. Therefore, the polynucleotide as defined herein may, without limitation, include single- and double-stranded DNAs, DNAs including single- and double-stranded regions, single- and double-stranded RNAs, and RNAs including single- and double-stranded regions, and hybrid molecules including DNAs and RNAs that may be single-stranded or more typically double-stranded or may include single- and double-stranded regions. Therefore, the DNA or RNA having a modified backbone due to its stability or other reasons corresponds to the "polynucleotide" as described in the terms intended herein. In addition, the DNA or RNA containing unusual bases such as inosine or modified bases such as a tritiated base is encompassed in the term "polynucleotide" as defined herein. Generally, the term "polynucleotide" includes all chemically, enzymatically and/or metabolically modified forms of an unmodified polynucleotide. The polynucleotide may be prepared by various methods including an in vitro recombinant DNA-mediated technology, and prepared by expression of DNA in cells and organisms, but the present disclosure is not limited thereto.

The kit of the present disclosure includes the composition of the present disclosure as described above, and is very economical because time and cost may be saved, compared to conventional gene search methods. In one embodiment of the present disclosure, a set of primers capable of detecting the expression level of various genes may be stacked together in one chip in the kit of the present disclosure, and thus time and cost may be saved compared to conventional methods.

The compositions and the kits to be used for diagnosing *a Helicobacter* pylori-associated disease according to the invention are as defined in claims 1 to 4.

### 2. A method for providing information necessary for diagnosis of a Helicobacter pylori-associated disease

Another aspect of the present disclosure provides a method for providing information necessary for diagnosis of a *Helicobacter* pylori-associated disease in a subject, the method comprising the steps of: preparing a specimen DNA from a sample of a subject; amplifying the specimen DNA using the composition or the kit of the present application; and determining the expression level of a gene whose expression is increased or decreased by *Helicobacter pylori* infection from the amplification result.

Another aspect of the present disclosure provides a method for providing information necessary for judging a difference in therapeutic effect in a subject infected with *Helicobacter pylori,* the method comprising the steps of: treating a subject, in which a gene whose expression is increased or decreased by *Helicobacter pylori* infection is identified, with any therapeutic candidate material for a *Helicobacter* pylori-associated disease or curing the subject by any method; and choosing any therapeutic candidate material or any method as a therapeutic candidate material or a therapeutic method, which is suitable for the patient, when the disease is ameliorated or treated with the any therapeutic candidate material or the any therapeutic method.

A still another aspect of the present disclosure provides a method for providing information necessary for diagnosis, for example, diagnosis of prognosis, of a *Helicobacter* pylori-associated disease in a patient infected with *Helicobacter pylori,* the method comprising the steps of: preparing a specimen DNA from a sample of a patient infected with *Helicobacter pylori;* amplifying the specimen DNA using the composition or the kit of the present disclosure; and determining the expression level of a gene whose expression is increased or decreased by *Helicobacter pylori* infection from the amplification result.

The description of the "composition and kit for diagnosing a *Helicobacter* pylori-associated disease" is the same as those described in **"1. A composition and a kit for diagnosing a *Helicobacter* pylori-associated disease,"** and thus, the detailed description thereof is omitted.

The any therapeutic candidate material may be a therapeutic agent generally used to treat a *Helicobacter* pylori-associated disease, or a novel material whose therapeutic effect against the disease is not known, but the present disclosure is not limited thereto. Whether or not the any therapeutic candidate material has a therapeutic effect on a certain group of patients may be determined by treating a patient infected with *Helicobacter pylori* with the any therapeutic candidate material to check the therapeutic effect. When the therapeutic candidate material has a therapeutic effect against gastritis caused by *Helicobacter pylori* infection, it may be predicted that the therapeutic candidate material has a high therapeutic effect when the therapeutic candidate material is applied to a group of patients having another disease caused by *Helicobacter pylori* infection, for example, gastric ulcer or gastric cancer, thereby providing useful information to determine a therapeutic strategy. In addition, when a therapeutic effect is not exerted against gastritis caused by *Helicobacter pylori* infection by the use of the any therapeutic candidate material, the unnecessary treatment needs not to be performed by suspending the therapy on the group of patients having another disease caused by *Helicobacter pylori* infection, for example, gastric ulcer or gastric cancer. Therefore, a therapeutic strategy may be effectively designed.

In the present disclosure, the term "sample" is meant to include, without limitation, any biological specimen obtained from a patient. In one embodiment of the present disclosure, the sample may include, without limitation, a fine needle aspirate obtained from a patient's stomach (for example, aspirate harvested by random mammary fine needle aspiration), a tissue sample (for example, gastritis, gastric ulcer, gastric cancer tissues), for example, a tumor biopsy (for example, an aspiration biopsy, a surgical resection of tumor), and cell extracts thereof. In one embodiment, the sample is, for example, a formalin-fixed paraffin-embedded (FFPE) tissue sample prepared from gastritis, gastric ulcer, or gastric cancer. In another embodiment of the present disclosure, the sample is a tissue lysate or extract prepared from a frozen tissue obtained from a subject having gastritis, gastric ulcer, or gastric cancer.

The "subject" of the present disclosure may be all animals, such as rats, mice, and livestock, including humans. Specifically, the subject may be one infected or suspected to be infected with *Helicobacter pylori.* As another example, the subject may be one having or likely to develop a *Helicobacter* pylori-associated disease. More specifically, the subject may be a patient infected with *Helicobacter pylori.*

In the present disclosure, the term "patient" is meant to typically include humans, but also include other animals such as other primates, rodents, canines, felines, equines, ovines, porcines, and the like.

In the present disclosure, the term "amelioration" refers to, without limitation, all actions that at least decrease parameters, such as a degree of symptom, associated with a treatment.

In the present disclosure, the term "prevention" is meant to include all actions that inhibit or delay a symptom of the *Helicobacter* pylori-associated disease.

In the present disclosure, the term "treatment" is mean to include all actions that improve or beneficially modify a symptom of the *Helicobacter* pylori-associated disease.

In addition, in the present disclosure, the term "administration" refers to introducing a predetermined substance into a subject in an appropriate manner.

In the present disclosure, the expression of the gene, whose expression is increased or decreased by *Helicobacter pylori* infection, disclosed in the present disclosure in a sample of a patient having a *Helicobacter* pylori-associated disease may be analyzed to determine what the prognosis of a subject having a target specimen is for the *Helicobacter* pylori-associated disease. In one embodiment of the present disclosure, the gene whose expression is increased by *Helicobacter pylori* infection may be selected from the group consisting of FGF21, CTH, CREBRF, DLL4, FGF18, FOS, PDK1, PTPRN, CLIC4, PTPRB, PPP1R15A, PTPRH, DDIT3, BIRC3, FOXO3, BCL2, PRKCE, CHMP4C, CLDN14, SLC7A11, BOC, AJUBA, LMO7, MMP24, C3orf58, KLF10, CSGALNACT1, CEP120, CHAC1, ASNS, NFE2L2, RIOK3, TXNIP, MTR, IFRD1, and a combination thereof, and specifically, may be selected from the group consisting of FGF21, CTH, CREBRF, PTPRN and a combination thereof, but the present application is not limited thereto. In another embodiment of the present disclosure, the gene whose expression is decreased by *Helicobacter pylori* infection may be selected from the group consisting of ADGRA2, TBX4, OVOL2, ACVRL1, ALB, JADE1, MLLT11, ADORA1, TNFRSF8, NLRP12, CASP14, LTA, SMAGP, NEO1, MTSS1, TSPAN32, CLDN8, MYBPH, SGCE, CDH15, ARHGAP6, ZFP36L2, EHF, SLAMF6, HLA-DPB1, SSC5D, CCR4, CCR7, KLRG1, BLNK, IGFBP1, GIF, and a combination thereof, but the present disclosure is not limited thereto.

The present inventors have first found that the increase or decrease in the expressions of the genes may be used as a diagnostic marker capable of predicting a difference in therapeutic effect for the patient having the *Helicobacter* pylori-associated disease or diagnosing the prognosis of the patient having the *Helicobacter* pylori-associated disease. In one embodiment of the present disclosure, the method for providing information necessary for diagnosis of prognosis of a *Helicobacter* pylori-associated disease of the present disclosure can be used in diagnosing the disease occurrence, increasing the survival rate, or reducing the relapse rate of the patient having the disease. In another embodiment of the present disclosure, through the method of the present application, the therapeutic effect against the disease may be predicted or the survival rate or the relapse rate of the patient having the disease may be predicted, and thus information for finding therapeutic agents suitable for each patient and selecting therapeutic methods can be provided. Accordingly, a therapeutic strategy for the *Helicobacter* pylori-associated disease can be efficiently designed.

The present disclosure provides methods for diagnosis and prognosis of a disease by analyzing changes in gene expression in vivo following *Helicobacter pylori* infection. In one embodiment of the present disclosure, classification allows optimization of treatment, and determination of whether on whether to proceed with a specific therapy, and how to optimize dose, choice of treatment, and the like. Analysis of expression patterns of a group of genes for single cell also provides for the identification and development of therapies which target mutations and/or pathways in disease-state cells.

In another embodiment of the present disclosure, a method for examining and analyzing the expression of one or more genetic markers in a tissue or cell sample in a mammal is provided. In one embodiment, the expression of one or more genetic markers indicates that a mammalian subject from which tissue or cell sample is collected is quite likely to have the *Helicobacter* pylori-associated disease.

The method for providing information necessary for diagnosis of a *Helicobacter* pylori-associated disease according to the invention is as defined in claims 5 to 7.

In one embodiment of the present disclosure, which is not part of the claimed invention, a symptom of the *Helicobacter* pylori-associated disease can be ameliorated or treated by letting the subject infected with *Helicobacter pylori* intake the kimchi including lactic acid bacteria or administering the kimchi including lactic acid bacteria to the subject. In one embodiment of the present disclosure, the kimchi may be, but is not limited to, kimchi including *Leuconostoc mesenteroides* CJLM119 and *Lactobacillus plantarum* CJLP133. The *Leuconostoc mesenteroides* CJLM119 is the microbial strain deposited at the Korea Research Institute of Bioscience and Biotechnology Gene Bank under the accession number KCTC 13043BP, and a method for isolation and identification of the strain is specifically disclosed in Korean Patent No. 1,807,995. In addition, the *Lactobacillus plantarum* CJLP133 is the microbial strain deposited at the Korea Research Institute of Bioscience and Biotechnology Gene Bank under the accession number KCTC 11403BP, and a method for isolation and identification of the strain is specifically disclosed in Korean Patent No. 1,486,999.

The "kimchi" is meant to include, without limitation, foods prepared by salting and seasoning vegetables and then fermenting the same. For example, the vegetable may be Kimchi cabbages, white radishes, spring onions, mustard leaves, cucumbers, and the like. Specifically, the vegetable may be Kimchi cabbages, and any other vegetables may be used as raw materials of kimchi. In one embodiment of the present disclosure, the vegetable may be, but is not limited to, Kimchi cabbages, and specifically Kimchi cabbages having a high content of lycopene.

In one embodiment of the present disclosure, which is not part of the claimed invention, the seasoning is prepared by mixing ingredients such as red pepper powders, garlic, ginger, salted fish, etc., and in addition to these, for the ingredients constituting the seasoning, any other ingredients can be added or excluded as appropriate, if necessary depending on personal preferences, fermentation methods, fermentation period, etc. The kimchi of the present disclosure may be prepared by a method of mixing the salted vegetables, for example, the salted Kimchi cabbages, with the seasoning and then fermenting the same, according to a conventional method for preparing kimchi. As the Chinese cabbage, it may be more appropriate to use Chinese cabbage having a high content of lycopene. However, the present disclosure is not limited thereto, and conventional Chinese cabbage may be used without limitation for the preparation of the kimchi.

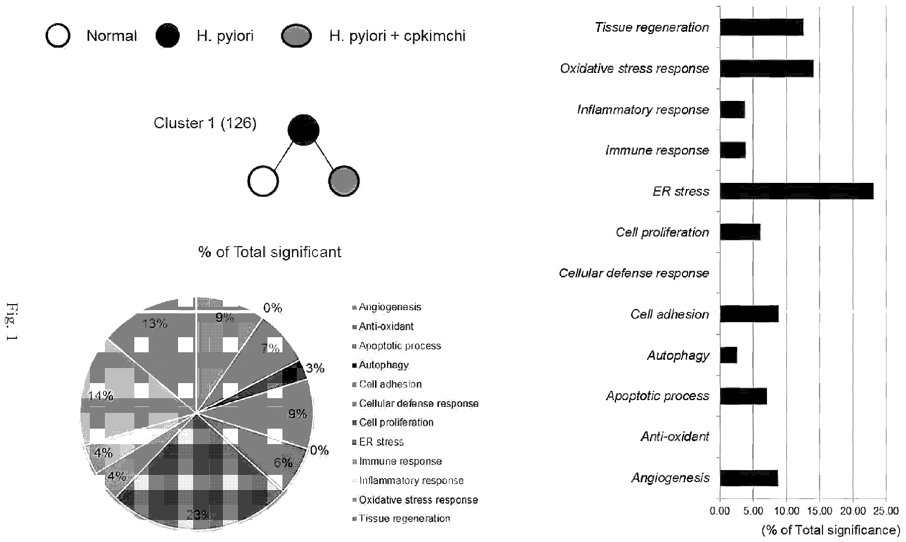

According to one embodiment of the present disclosure, the expressions of genes such as genes for ER stress, genes for oxidative stress, genes for tissue regeneration, genes for angiogenesis, and the like may be increased by *Helicobacter pylori* infection *in vitro* or *in vivo* (see FIG. 1, FIG. 2, and FIG. 5).

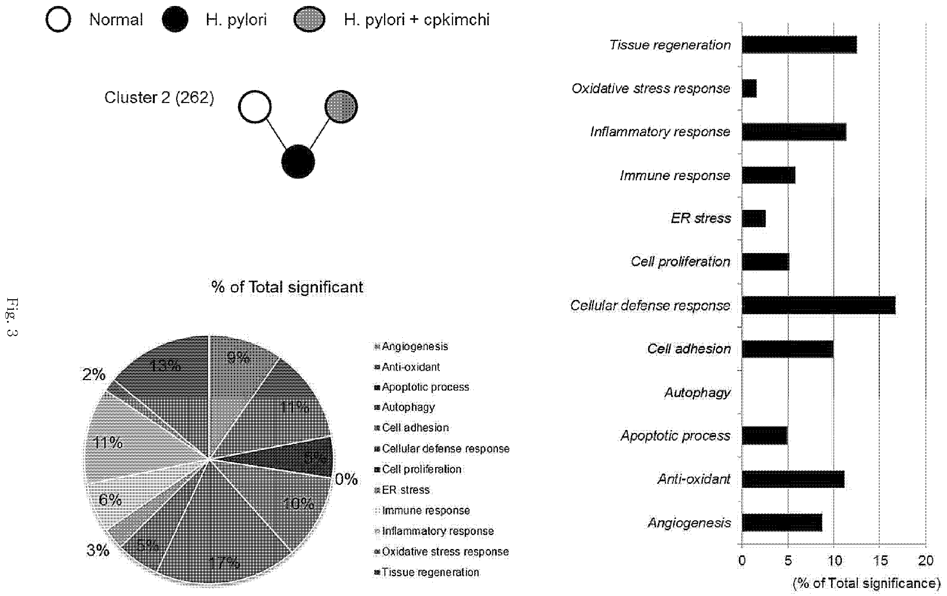

According to another embodiment of the present disclosure, the expressions of genes such as genes for cellular defense response, genes for tissue regeneration, genes for antioxidation, genes for cell adhesion, and the like may be decreased by *Helicobacter pylori* infection *in vitro* or in *vivo* (see FIG. 3, FIG. 4, and FIG. 6).

As such, a list of the genes whose expressions are increased or decreased by *Helicobacter pylori* infection, identified in the present disclosure, may be usefully used for the prevention, treatment, or amelioration of various diseases that may be caused by *Helicobacter pylori,* for example, gastritis, gastric ulcer, and gastric cancer.

Hereinafter, the present invention will be described in detail through Examples and Experimental examples.

However, the following Examples and Experimental examples merely illustrate the present invention, and the contents of the present invention are not limited by the following Examples and Experimental examples.

In the following Examples and Experimental examples, statistical analyses were conducted with GraphPad Prism (GraphPad Software, La Jolla, CA, USA) and SPSS software (version 12.0; SPSS Inc., Chicago, IL, USA). Statistical significance between groups was determined by Mann-Whitney U test. Statistical significance was accepted at p < 0.05.

### Example 1: Preparation of Helicobacter pylori-infected gastric cancer cells and animal models

### <1-1> Helicobacter pylori-infected gastric cancer cell model

The gastric adenocarcinoma cell line (AGS) was purchased from ATCC (Manassas, VA) and stored and used. All cells were not used for more than 6 months after resuscitation. AGS cells were cultured at 37°C, 5% CO₂ in a medium containing RPMI-1640 culture solution (Gibco BRL, Gaithersburg, MD) and 10% fetal bovine serum (FBS, Gibco BRL). Cells were dispensed in a 6-well plate at a concentration of 10⁵ cells/ml, adsorbed for 24 hours, and then exposed to *Helicobacter pylori* at 50 MOI (500⁵ CFU) for 6 hours to infect the cells.

### <1-2> Preparation of anticancer kimchi

Kimchi was prepared according to a conventional kimchi preparation method. Anticancer kimchi (hereinafter, sometimes referred to as 'CpKimchi' or 'CPK') was prepared by the same method for preparation of standard kimchi (hereinafter, sometimes referred to as 'sKimchi' or 'SK'), except that: as a main material, the Chinese cabbage having a high content of lycopene was used instead of general Chinese cabbage, and two kinds of lactic acid bacteria, the *Leuconostoc mesenteroides* CJLM119 and the *Lactobacillus plantarum* CJLP133 were used. The *Leuconostoc mesenteroides* CJLM119 is the microbial strain deposited at the Korea Research Institute of Bioscience and Biotechnology Gene Bank under the accession number KCTC 13043BP, and a method for isolation and identification of the strain is specifically disclosed in Korean Patent No. 10-1807995. In addition, the *Lactobacillus plantarum* CJLP133 is the microbial strain deposited at the Korea Research Institute of Bioscience and Biotechnology Gene Bank under the accession number KCTC 11403BP, and a method for isolation and identification of the strain is specifically disclosed in Korean Patent No. 10-1486999. Accordingly, the detailed description thereof is omitted.

### <1-3> Preparation of concentrate of kimchi extract

The kimchi prepared in Example <1-2> was freeze-dried and then ground into powders. To the powders, 20 times the weight of the powders of methanol was added, and then, extraction was performed with a stirrer for 12 hours. The extracted supernatant was filtered through a filter paper, and methanol was again added to the remaining precipitate to perform extraction for 12 hours. The extract was thermally concentrated at a temperature of about 40°C by a concentrator to prepare a concentrate, and the concentrate was stored at 4°C for use in further experiments.

### <1-4> Preparation for in vitro and in vivo experimental models

For *in vitro* experiment, the concentrate of the kimchi extract prepared in Example <1-3> was used at 5 mg/ml and 10 mg /mℓ, respectively. The kimchi intake for *in vivo* experiment was set as follows: The usual kimchi intake for Korean is about 30 to 100 g per day depending on personal preference, and the concentrate of the anticancer kimchi extract was mixed with the dietary pellets of animal models once a week, twice the weight, and at the ratios of 1.7 g/kg/day, and 5.1 g/kg/day, and the resulting mixture was converted into the usual kimchi intake for Korean and used.

### <1-5> RNA isolation and RT-PCR performance

The culture medium of *Helicobacter* pylori-infected gastric cancer cells prepared in Example <1-1> was removed by suction, and then the cells were washed twice with Dulbecco's PBS. RiboEX (500 µl, GeneAll, Seoul, South Korea) was added to a plate incubated at 4°C for 10 minutes. RiboEX was collected and transferred into a 1.5 ml tube, and 100 µl of chloroform was added and mixed slowly. After standing for 10 minutes on ice, samples were centrifuged at 10,000 g for 30 minutes. The supernatant was mixed with 200 µl of isopropanol, and then the mixture was left at 4°C for 1 hour. After centrifugation at 13,000 g for 30 minutes, the precipitate was washed with 70% (vol/vol) ethanol. Ethanol was completely evaporated, and then the precipitate was dissolved in 100 µl of water treated with diethylene pyrocarbonate (DEPC) (Invitrogen Life Technologies, Carlsbad, CA). cDNA was prepared using the reverse transcriptase enzyme derived from Moroni murine leukemia virus (Promega, Madison, WI). PCR was performed using 30 cycles of 20 seconds at 94°C, 30 seconds at 58°C, and 45 seconds at 72°C.

### <1-6> mRNA sequencing

A library was prepared from 2 µg total RNA using the SMARTer Stranded RNA-Seq kit (Clontech Laboratories, Inc., USA). Isolation of mRNA was performed using Poly (A) RNA SelectionKit (LEXOGEN, Inc., Austria). The isolated mRNA was used for cDNA synthesis and cleavage. Indexing was done using Illumina indices 1 to 12 and amplification was performed by PCR. Then the average size of fragments was assessed by checking the library with Agilent 2100 bioanalyzer (DNA High Sensitivity Kit). For quantification, a library quantification kit using Step One real time PCR system (Life Technologies, Inc., USA) was used. High-throughput sequencing was performed by paired-end 100 sequencing using HiSeq 2500 (Illumina, Inc., USA).

### <1-7> Data analysis

For mRNA-Seq analysis, TopHat software (Cole Trapnell et al., 2009) tool was used in order to obtain the alignment file. Differentially expressed genes were determined based on counts from unique and multiple alignments using the coverage of Bedtools (Quinlan AR, 2010). The RT (Read Count) data were processed based on Quantile normalization method using EdgeR within statistical program R (R development Core Team, 2016) using Bioconductor (Gentleman et al., 2004). The alignment files also were used for assembling transcripts, estimating their abundances and detecting the differential expression of genes or isoforms using Cufflinks. Fragments per kilobase of exon per million fragments (FPKM) were used to determine the expression levels of the gene regions. Gene classification was based on the results of searches performed with DAVID (http://david.abcc.ncifcrf.gov/).

### Example 2: Genes that are specifically increased following Helicobacter pylori infection

### <2-1> Identification of genes through RNAseq

RNAseq analysis was performed in order to find *Helicobacter pylori* infection-induced target genes, and among genes, 126 genes, whose expressions were increased by *Helicobacter pylori* infection, but were normalized by combination treatment with CpKimchi, were identified. These genes were found to be genes for ER stress, genes for oxidative stress, genes for tissue regeneration, genes for angiogenesis, etc. (FIG. 1).

### <2-2> Validation by RT-PCR of the RNAseq result

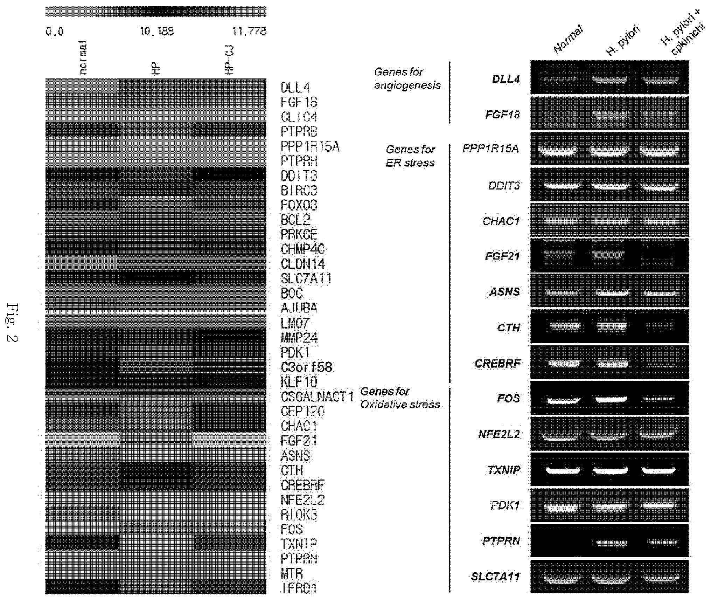

The RNAseq heatmap analysis result was verified by RT-PCR. Among the genes for angiogenesis, the expressions of DLL4 (delta-like 4) and FGF18 (fibroblast growth factor 18) were significantly decreased following the combination treatment with CpKimchi. The expressions of FGF21 (fibroblast growth factor 21), CTH (cystathionine-lyase) and CREBPF (cAMP responsive element binding protein), among the genes for ER stress, and the expressions of FOS, PDK1 (phosphoinositide-dependent kinase-1) and PTPRN (receptor-type tyrosine-protein phosphatase-like N), among the genes for oxidative stress, were also significantly decreased following the combination treatment with CpKimchi (FIG. 2).

### Example 3: Genes whose expressions are decreased by Helicobacter pylori infection

### <3-1> Identification of genes through RNAseq

Among genes, the genes, whose expressions were decreased by *Helicobacter pylori* infection, but were normalized and thus increased by treatment with CpKimchi, were analyzed. A total of 262 genes were identified. The genes, whose expressions were decreased by *Helicobacter pylori* infection, but were normalized by combination treatment with CpKimchi, were classified into genes for cellular defense response, genes for tissue regeneration, genes for antioxidation, genes for cell adhesion, etc. (FIG. 3).

### <3-2> Validation by RT-PCR of the RNAseq result

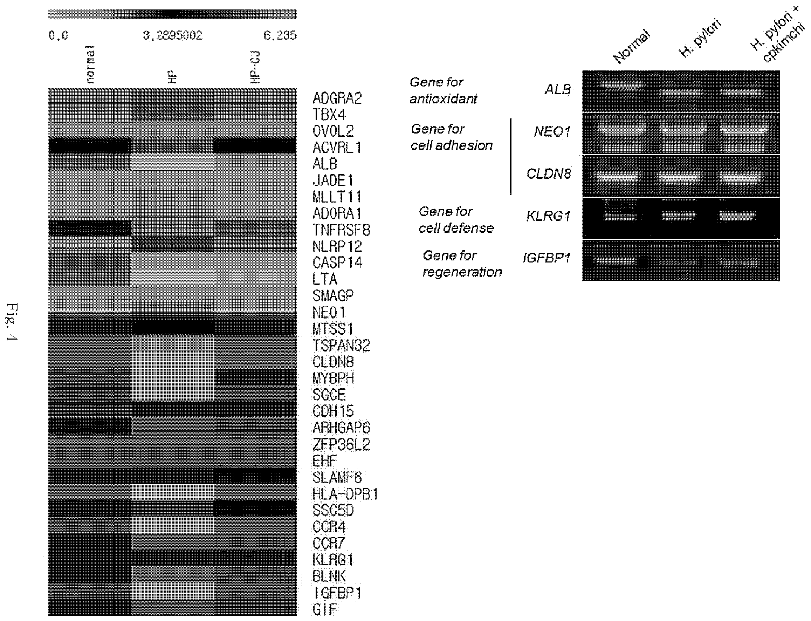

The RNAseq heatmap analysis result was verified by RT-PCR. For ALB, NEO1 (neogenin precursor1), CLDN8 (claudin 8), KLRG1 (killer cell lectin-like receptor subfamily G member 1) and IGFBP1 (insulin-like growth factor-binding protein 1), it was found that the expressions of genes which were decreased following the treatment with CpKimchi were normalized (FIG. 4) .

### Example 4: In vivo verification of genes that are specifically increased by Helicobacter pylori infection

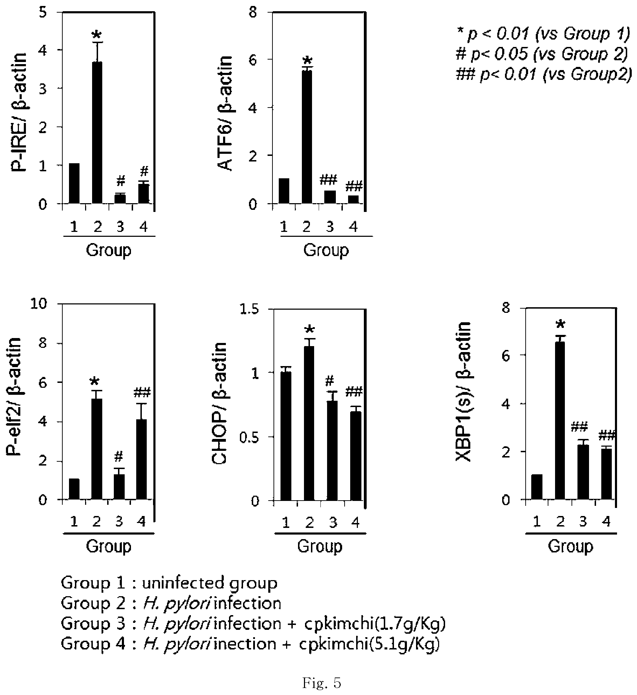

In the *Helicobacter pylori* infection animal model experiment, the expressions of the genes for ER stress, that were the same as the RNAseq result, were identified. In group 2 infected with *Helicobacter pylori,* the expressions of the genes for ER stress were significantly increased, which is consistent with the RNAseq result. These genes involved in ER stress were significantly improved in CpKimchi-administered groups 3 and 4, which is also consistent with the RNAseq result (FIG. 5).

### Example 5: In vivo verification of genes that are specifically decreased by Helicobacter pylori infection

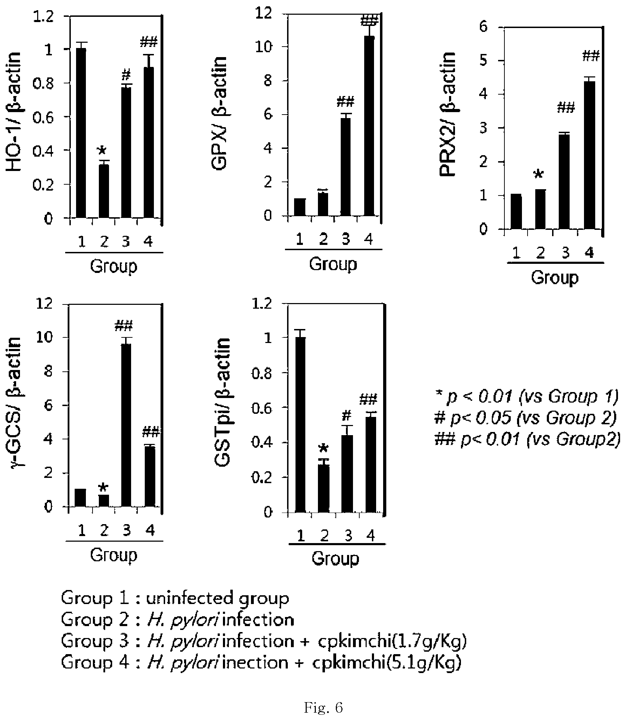

In the *Helicobacter pylori* infection animal model, the expressions of the genes for antioxidation, that were the same as the RNAseq result, were identified. The expressions of the genes for antioxidation were significantly decreased in *Helicobacter* pylori-infected group 2, but were significantly increased and restored in both CpKimchi-administered groups 3 and 4 (Fig. 6).

Although exemplary embodiments of the present disclosure have been described above, the scope of the present disclosure is not limited to the specific embodiments as described above, a person skilled in the art can change the present disclosure within the scope described in the claims of the present application.

## Claims

1. Use of a composition for *in vitro* diagnosis of a *Helicobacter* pylori-associated disease, wherein the composition comprises a preparation for measuring the expression amount of FGF21 (fibroblast growth factor 21, Gene ID: 26291) gene, said preparation comprising a probe or a primer specifically binding to the mRNA or cDNA of said FGF21 gene, or an antibody or an aptamer specifically binding to the protein expressed from said FGF21 gene.

2. Use according to claim 1, wherein the composition further comprises a preparation for measuring the expression amount of CTH (cystathionine g-lyase, Gene ID: 1491) or CREBRF (cAMP responsive element binding protein, Gene ID: 153222) genes, and combination thereof, said preparation comprising a probe or a primer specifically binding to the mRNA or cDNA of one of said genes, or an antibody or an aptamer specifically binding to the protein expressed from one of said genes.

3. Use according to claim 2, wherein the composition further comprises a preparation for measuring the expression amount of a gene selected from the group consisting of: DLL4 (delta-like 4, Gene ID: 54567), FGF18 (fibroblast growth factor 18, Gene ID: 8817), FOS (Fos proto-oncogene, AP-1 transcription factor subunit, Gene ID: 2353), PDK1 (phosphoinositide-dependent kinase-1, Gene ID: 5170), PTPRN (receptor-type tyrosine-protein phosphatase-like N, Gene ID: 5798), CLIC4 (chloride intracellular channel 4, Gene ID: 25932), PTPRB (protein tyrosine phosphatase, receptor type B, Gene ID: 5787), PPP1R15A (protein phosphatase 1 regulatory subunit 15A, Gene ID: 23645), PTPRH (protein tyrosine phosphatase, receptor type H, Gene ID: 5794), DDIT3 (DNA damage inducible transcript 3, Gene ID: 1649), BIRC3 (baculoviral IAP repeat containing 3, Gene ID: 330), FOXO3 (forkhead box O3, Gene ID: 2309), BCL2 (BCL2, apoptosis regulator, Gene ID: 596), PRKCE (protein kinase C epsilon, Gene ID: 5581), CHMP4C (charged multivesicular body protein 4C, Gene ID: 92421), CLDN14 (claudin 14, Gene ID: 23562), SLC7A11 (solute carrier family 7 member 11, Gene ID: 23657), BOC (BOC cell adhesion associated, oncogene regulated, Gene ID: 91653), AJUBA (ajuba LIM protein, Gene ID: 84962), LMO7 (LIM domain 7, Gene ID: 4008), MMP24 (matrix metallopeptidase 24, Gene ID: 10893), C3orf58 (divergent protein kinase domain 2A, Gene ID: 205428), KLF10 (Kruppel like factor 10, Gene ID: 7071), CSGALNACT1 (chondroitin sulfate N-acetylgalactosaminyltransferase 1, Gene ID: 55790), CEP120 (centrosomal protein 120, Gene ID: 153241), CHAC1 (ChaC glutathione specific gamma-glutamylcyclotransferase 1, Gene ID: 79094), ASNS (asparagine synthetase (glutamine-hydrolyzing), Gene ID: 440), NFE2L2 (nuclear factor, erythroid 2 like 2, Gene ID: 4780), RIOK3 (RIO kinase 3, Gene ID: 8780), TXNIP (thioredoxin interacting protein, Gene ID: 10628), MTR (5-methyltetrahydrofolate-homocysteine methyltransferase, Gene ID: 4548), IFRD1 (interferon related developmental regulator 1, Gene ID: 3475), ADGRA2 (adhesion G protein-coupled receptor A2, Gene ID: 25960), TBX4 (T-box 4, Gene ID: 9496), OVOL2 (ovo like zinc finger 2, Gene ID: 58495), ACVRL1 (activin A receptor like type 1, Gene ID: 94), ALB (albumin, Gene ID: 213), JADE1 (jade family PHD finger 1, Gene ID: 79960), MLLT11 (MLLT11, transcription factor 7 cofactor, Gene ID: 10962), ADORA1 (adenosine A1 receptor, Gene ID: 134), TNFRSF8 (TNF receptor superfamily member 8, Gene ID: 943), NLRP12 (NLR family pyrin domain containing 12, Gene ID: 91662), CASP14 (caspase 14, Gene ID: 23581), LTA (lymphotoxin alpha, Gene ID: 4049), SMAGP (small cell adhesion glycoprotein, Gene ID: 57228), NEO1 (neogenin precursor1, Gene ID: 4756), MTSS1 (MTSS1, I-BAR domain containing, Gene ID: 9788), TSPAN32 (tetraspanin 32, Gene ID: 10077), CLDN8 (claudin 8, Gene ID: 9073), MYBPH (myosin binding protein H, Gene ID: 4608), SGCE (sarcoglycan epsilon, Gene ID: 8910), CDH15 (cadherin 15, Gene ID: 1013), ARHGAP6 (Rho GTPase activating protein 6, Gene ID: 395), ZFP36L2 (ZFP36 ring finger protein like 2, Gene ID: 678), EHF (ETS homologous factor, Gene ID: 26298), SLAMF6 (SLAM family member 6, Gene ID: 114836), HLA-DPB1 (major histocompatibility complex, class II, DP beta 1, Gene ID: 3115), SSC5D (scavenger receptor cysteine rich family member with 5 domains, Gene ID: 284297), CCR4 (C-C motif chemokine receptor 4, Gene ID: 1233), CCR7 (C-C motif chemokine receptor 7, Gene ID: 1236), KLRG1 (killer cell lectin-like receptor subfamily G member 1, Gene ID: 10219), BLNK (B cell linker, Gene ID: 29760), IGFBP1 (insulin-like growth factor=binding protein 1, Gene ID: 3484), GIF (MIF, macrophage migration inhibitory factor, Gene ID: 4282) genes, and a combination thereof, said preparation comprising a probe or a primer specifically binding to the mRNA or cDNA of one of said genes, or an antibody or an aptamer specifically binding to the protein expressed from one of said genes.

4. Use of a kit for *in vitro* diagnosis of a *Helicobacter* pylori-associated disease, wherein the kit comprises the composition as defined in any of claims 1 to 3.

5. A method for providing information necessary for diagnosis of a *Helicobacter* pylori-associated disease in a patient infected with *Helicobacter pylori,* the method comprising the step of determining the expression level of a gene whose expression is increased or decreased by *Helicobacter pylori* infection in a sample of the patient infected with Helicobacter pylori, wherein the step of determining the expression level of the gene comprises measuring the expression amount of FGF21 (fibroblast growth factor 21, Gene ID: 26291) gene using a probe or a primer specifically binding to the mRNA or cDNA of said FGF21 gene, or an antibody or an aptamer specifically binding to the protein expressed from said FGF21 gene.

6. The method for providing information necessary for diagnosis of a *Helicobacter* pylori-associated disease of claim 5, wherein the step of determining the expression level of the gene further comprises measuring the expression amount of CTH (cystathionine g-lyase, Gene ID: 1491) or CREBRF (cAMP responsive element binding protein, Gene ID: 153222) genes, and combination thereof, said preparation comprising a probe or a primer specifically binding to the mRNA or cDNA of one of said genes, or an antibody or an aptamer specifically binding to the protein expressed from one of said genes.

7. The method for providing information necessary for diagnosis of a *Helicobacter* pylori-associated disease of claim 6, wherein the step of determining the expression level of the gene further comprises measuring the expression amount of a gene selected from the group consisting of: DLL4 (delta-like 4, Gene ID: 54567), FGF18 (fibroblast growth factor 18, Gene ID: 8817), FOS (Fos proto-oncogene, AP-1 transcription factor subunit, Gene ID: 2353), PDK1 (phosphoinositide-dependent kinase-1, Gene ID: 5170), PTPRN (receptor-type tyrosine-protein phosphatase-like N, Gene ID: 5798), CLIC4 (chloride intracellular channel 4, Gene ID: 25932), PTPRB (protein tyrosine phosphatase, receptor type B, Gene ID: 5787), PPP1R15A (protein phosphatase 1 regulatory subunit 15A, Gene ID: 23645), PTPRH (protein tyrosine phosphatase, receptor type H, Gene ID: 5794), DDIT3 (DNA damage inducible transcript 3, Gene ID: 1649), BIRC3 (baculoviral IAP repeat containing 3, Gene ID: 330), FOXO3 (forkhead box O3, Gene ID: 2309), BCL2 (BCL2, apoptosis regulator, Gene ID: 596), PRKCE (protein kinase C epsilon, Gene ID: 5581), CHMP4C (charged multivesicular body protein 4C, Gene ID: 92421), CLDN14 (claudin 14, Gene ID: 23562), SLC7A11 (solute carrier family 7 member 11, Gene ID: 23657), BOC (BOC cell adhesion associated, oncogene regulated, Gene ID: 91653), AJUBA (ajuba LIM protein, Gene ID: 84962), LMO7 (LIM domain 7, Gene ID: 4008), MMP24 (matrix metallopeptidase 24, Gene ID: 10893), C3orf58 (divergent protein kinase domain 2A, Gene ID: 205428), KLF10 (Kruppel like factor 10, Gene ID: 7071), CSGALNACT1 (chondroitin sulfate N-acetylgalactosaminyltransferase 1, Gene ID: 55790), CEP120 (centrosomal protein 120, Gene ID: 153241), CHAC1 (ChaC glutathione specific gamma-glutamylcyclotransferase 1, Gene ID: 79094), ASNS (asparagine synthetase (glutamine-hydrolyzing), Gene ID: 440), NFE2L2 (nuclear factor, erythroid 2 like 2, Gene ID: 4780), RIOK3 (RIO kinase 3, Gene ID: 8780), TXNIP (thioredoxin interacting protein, Gene ID: 10628), MTR (5-methyltetrahydrofolate-homocysteine methyltransferase, Gene ID: 4548), IFRD1 (interferon related developmental regulator 1, Gene ID: 3475), ADGRA2 (adhesion G protein-coupled receptor A2, Gene ID: 25960), TBX4 (T-box 4, Gene ID: 9496), OVOL2 (ovo like zinc finger 2, Gene ID: 58495), ACVRL1 (activin A receptor like type 1, Gene ID: 94), ALB (albumin, Gene ID: 213), JADE1 (jade family PHD finger 1, Gene ID: 79960), MLLT11 (MLLT11, transcription factor 7 cofactor, Gene ID: 10962), ADORA1 (adenosine A1 receptor, Gene ID: 134), TNFRSF8 (TNF receptor superfamily member 8, Gene ID: 943), NLRP12 (NLR family pyrin domain containing 12, Gene ID: 91662), CASP14 (caspase 14, Gene ID: 23581), LTA (lymphotoxin alpha, Gene ID: 4049), SMAGP (small cell adhesion glycoprotein, Gene ID: 57228), NEO1 (neogenin precursor1, Gene ID: 4756), MTSS1 (MTSS1, I-BAR domain containing, Gene ID: 9788), TSPAN32 (tetraspanin 32, Gene ID: 10077), CLDN8 (claudin 8, Gene ID: 9073), MYBPH (myosin binding protein H, Gene ID: 4608), SGCE (sarcoglycan epsilon, Gene ID: 8910), CDH15 (cadherin 15, Gene ID: 1013), ARHGAP6 (Rho GTPase activating protein 6, Gene ID: 395), ZFP36L2 (ZFP36 ring finger protein like 2, Gene ID: 678), EHF (ETS homologous factor, Gene ID: 26298), SLAMF6 (SLAM family member 6, Gene ID: 114836), HLA-DPB1 (major histocompatibility complex, class II, DP beta 1, Gene ID: 3115), SSC5D (scavenger receptor cysteine rich family member with 5 domains, Gene ID: 284297), CCR4 (C-C motif chemokine receptor 4, Gene ID: 1233), CCR7 (C-C motif chemokine receptor 7, Gene ID: 1236), KLRG1 (killer cell lectin-like receptor subfamily G member 1, Gene ID: 10219), BLNK (B cell linker, Gene ID: 29760), IGFBP1 (insulin-like growth factor=binding protein 1, Gene ID: 3484), GIF (MIF, macrophage migration inhibitory factor, Gene ID: 4282) genes, and a combination thereof, said preparation comprising a probe or a primer specifically binding to the mRNA or cDNA of one of said genes, or an antibody or an aptamer specifically binding to the protein expressed from one of said genes.

## Patentansprüche

1. Verwendung einer Zusammensetzung zur *in-vitro*-Diagnose einer *Helicobacter-pylori-assoziierten* Erkrankung, wobei die Zusammensetzung eine Zubereitung zur Messung der exprimierten Menge des Gens FGF21 (Fibroblastenwachstumsfaktor 21, Gen-ID: 26291) umfasst, wobei die Zubereitung eine Sonde oder einen Primer, die/der auf spezifische Weise an die mRNA oder cDNA des FGF21-Gens bindet, oder einen Antikörper oder ein Aptamer umfasst, der/das auf spezifische Weise an das Protein bindet, welches ausgehend von dem FGF21-Gen exprimiert wird.

2. Verwendung gemäß Anspruch 1, wobei die Zusammensetzung ferner eine Zubereitung zur Messung der exprimierten Menge der Gene CTH (Cystathionin-g-Lyase, Gen-ID: 1491) oder CREBRF (cAMP Responsive Element Binding Protein, Gen-ID: 153222) und von deren Kombinationen umfasst, wobei die Zubereitung eine Sonde oder einen Primer, die/der auf spezifische Weise an die mRNA oder cDNA eines der Gene bindet, oder einen Antikörper oder ein Aptamer umfasst, der/das auf spezifische Weise an das Protein bindet, welches ausgehend einem der Gene exprimiert wird.

3. Verwendung gemäß Anspruch 2, wobei die Zusammensetzung ferner eine Zubereitung zur Messung der exprimierten Menge eines Gens umfasst, das aus der Gruppe ausgewählt wurde, welche aus den folgenden Genen besteht: DLL4 (delta-ähnliches Element 4, Gen-ID: 54567), FGF18 (Fibroblastenwachstumsfaktor 18, Gen-ID: 8817), FOS (Fos Proto-Onkogen, AP-1-Transkriptionsfaktor-Untereinheit, Gen-ID: T2353), PDK1 (Phosphoinositid-abhängige Kinase-1, Gen-ID: 5170), PTPRN (Tyrosin-Protein-Phosphatase-ähnliches Element N vom Rezeptortyp, Gen-ID: 5798), CLIC4 (intrazellulärer Chloridkanal 4, Gen-ID: 25932), PTPRB (Protein-Tyrosinphosphatase, Rezeptortyp B, Gen-ID: 5787), PPP1R15A (regulatorische Untereinheit 15A der Proteinphosphatase 1, Gen-ID: 23645), PTPRH (Protein-Tyrosinphosphatase, Rezeptortyp H, Gen-ID: 5794), DDIT3 (durch DNA-Schäden induzierbares Transkript 3, Gen-ID: 1649), BIRC3 (Baculovirales LAP-Wiederholelement mit 3 Einheiten, Gen-ID: 330), FOXO3 (Forkhead-Box O3, Gen-ID: 2309), BCL2 (BCL2, Apoptose-Regulator, Gen-ID: 596), PRKCE (Proteinkinase-C epsilon, Gen-ID: 5581), CHMP4C (geladenes multivesikuläres Körperprotein 4C, Gen-ID: 92421), CLDN14 (Claudin 14, Gen-ID: 23562), SLC7A11 (Mitglied 11 der Trägerfamilie für gelöste Stoffe 7, Gen-ID: 23657), BOC (onkogen regulierter, zelladhäsionsassoziierter BOC, Gen-ID: 91653), AJUBA (Ajuba LIM-Protein, Gen-ID: 84962), LMO7 (LIM-Domäne 7, Gen-ID: 4008), MMP24 (Matrix-Metallopeptidase 24, Gen-ID: 10893), C3orf58 (divergente Proteinkinase-Domäne 2A, Gen-ID: 205428), KLF10 (Kruppel-ähnlicher Faktor 10, Gen-ID: 7071), CSGALNACT1 (Chondroitinsulfat-N-acetylgalactosaminyltransferase 1, Gen-ID: 55790), CEP120 (centrosomales Protein 120, Gen-ID: 153241), CHAC1 (ChaC-Glutathion-spezifische gamma-Glutamylcyclotransferase 1, Gen-ID: 79094), ASN (Asparaginsynthetase (Glutamin-hydrolysierend), Gen-ID: 440), NFE2L2 (Kernfaktor, Erythroid-2-ähnliches Element 2, Gen-ID: 4780), RIOK3 (RIO-Kinase 3, Gen-ID: 8780), TXNIP (mit Thioredoxin interagierendes Protein, Gen-ID: 10628), MTR (5-Methyltetrahydrofolat-Homocystein-Methyltransferase, Gen-ID: 4548), IFRD1 (mit Interferon in Beziehung stehender Entwicklungsregulator 1, Gen-ID: 3475), ADGRA2 (Adhäsion-G-Protein-gekoppelter Rezeptor A2, Gen-ID: 25960), TBX4 (T-Box 4, Gen-ID: 9496), OVOL2 (Ovo-ähnlicher Zinkfinger 2, Gen-ID: 58495), ACVRL1 (Aktivin-A-Rezeptor-ähnliches Element Typ 1, Gen-ID: 94), ALB (Albumin, Gen-ID: 213), JADE1 (PHD-Finger 1 der Jade-Familie, Gen-ID: 79960), MLLT11 (MLLT11, co-Faktor des Transkriptionsfaktors 7, Gen-ID: 10962), ADORA1 (Adenosin-Al-Rezeptor, Gen-ID: 134), TNFRSF8 (Mitglied 8 der TNF-Rezeptor-Superfamilie, Gen-ID: 943), NLRP12 (NLR-Familie, pyrindomänenhaltig 12, Gen-ID: 91662), CASP14 (Caspase 14, Gen-ID: 23581), LTA (Lymphotoxin alpha, Gen-ID: 4049), SMAGP (Kleinzelladhäsions-Glycoprotein, Gen-ID: 57228), NEO1 (Neogenin-Vorläufer, Gen-ID: 4756), MTSS1 (MTSS1, I-BAR-domänenhaltig, Gen-ID: 9788), TSPAN32 (Tetraspanin 32, Gen-ID: 10077), CLDN8 (Claudin 8, Gen-ID: 9073), MYBPH (myosinbindendes Protein H, Gen-ID: 4608), SGCE (Sarkoglykan epsilon, Gen-ID: 8910), CDH15 (Cadherin 15, Gen-ID: 1013), ARHGAP6 (Rho-GTPase-aktivierendes Protein 6, Gen-ID: 395), ZFP36L2 (ZFP36 Ringfingerprotein-ähnliches Element 2, Gen-ID: 678), EHF (ETShomologer Faktor, Gen-ID: 26298), SLAMF6 (Mitglied 6 der SLAM-Familie, Gen-ID: 114836), HLA-DPB1 (Haupthistokompatibilitätskomplex, Klasse II, DP Beta 1, Gen-ID: 3115), SSC5D (Mitglied der cysteinreichen Scavenger-Rezeptoren mit 5 Domänen, Gen-ID: 284297), CCR4 (C-C-Motiv-Chemokinrezeptor 4, Gen-ID: 1233), CCR7 (C-C-Motiv-Chemokinrezeptor 7, Gen-ID: 1236), KLRG1 (Mitglied 1 der Unterfamilie G der Killerzellen-Lektin-ähnlichen Rezeptoren, Gen-ID: 10219), BLNK (B-Zelllinker, Gen-ID: 29760), IGFBP1 (Insulin-ähnliches Wachstumsfaktor-Bindungsprotein 1, Gen-ID: 3484), GIF (MIF, Makrophagen-Migrationshemmungsfaktor, Gen-ID: 4282) und einer Kombination davon, wobei die Zubereitung eine Sonde oder einen Primer, die/der auf spezifische Weise an die mRNA oder cDNA eines dieser Gene bindet, oder einen Antikörper oder ein Aptamer umfasst, der/das auf spezifische Weise an das Protein bindet, welches ausgehend von einem dieser Gene exprimiert wird.

4. Verwendung eines Kits zur *in-vitro*-Diagnose einer *Helicobacter-pylori-*assoziierten Erkrankung, wobei das Kit die Zusammensetzung gemäß der Definition in einem beliebigen der Ansprüche 1 bis 3 umfasst.

5. Verfahren zur Bereitstellung von Informationen, welche für die Diagnose einer *Helicobacter-pylori*-assoziierten Erkrankung bei einem mit *Helicobacter pylori* infizierten Patienten erforderlich sind, wobei das Verfahren den Schritt des Bestimmens des Expressionsniveaus eines Gens, dessen Expression durch eine *Helicobacter-pylori*-Infektion erhöht oder vermindert wird, in einer Probe umfasst, welche von dem mit *Helicobacter pylori* infizierten Patienten stammt, wobei der Schritt des Bestimmens des Expressionsniveaus des Gens die Messung der exprimierten Menge des Gens FGF21 (Fibroblastenwachstumsfaktor 21, Gen-ID: 26291) unter Verwendung einer Sonde oder eines Primers, die/der auf spezifische Weise an die mRNA oder cDNA des FGF21-Gens bindet, oder eines Antikörpers oder Aptamers umfasst, der/das auf spezifische Weise an das Protein bindet, welches ausgehend von dem FGF21-Gen exprimiert wird.

6. Verfahren zur Bereitstellung von Informationen, welche für eine Diagnose einer *Helicobacter-pylori-*assoziierten Erkrankung nach Anspruch 5 erforderlich sind, wobei der Schritt des Bestimmens des Expressionsniveaus des Gens ferner ein Messen der exprimierten Menge der Gene CTH (Cystathionin-g-Lyase, Gen-ID: 1491) oder CREBRF (cAMP Responsive Element Binding Protein, Gen-ID: 153222) und von deren Kombinationen umfasst, wobei die Zubereitung eine Sonde oder einen Primer, die/der auf spezifische Weise an die mRNA oder cDNA eines der Gene bindet, oder einen Antikörper oder ein Aptamer umfasst, der/das auf spezifische Weise an das Protein bindet, welches ausgehend einem der Gene exprimiert wird.

7. Verfahren zur Bereitstellung von Informationen, welche für die Diagnose einer *Helicobacter-pylori-*assoziierten Erkrankung nach Anspruch 6 erforderlich sind, wobei der Schritt des Bestimmens des Expressionsniveaus des Gens ferner ein Messen der exprimierten Menge eines Gens umfasst, das aus der Gruppe ausgewählt ist, welche aus den folgenden Genen besteht: DLL4 (delta-ähnliches Element 4, Gen-ID: 54567), FGF18 (Fibroblastenwachstumsfaktor 18, Gen-ID: 8817), FOS (Fos Proto-Onkogen, AP-1-Transkriptionsfaktor-Untereinheit, Gen-ID: T2353), PDK1 (Phosphoinositid-abhängige Kinase-1, Gen-ID: 5170), PTPRN (Tyrosin-Protein-Phosphatase-ähnliches Element N vom Rezeptortyp, Gen-ID: 5798), CLIC4 (intrazellulärer Chloridkanal 4, Gen-ID: 25932), PTPRB (Protein-Tyrosinphosphatase, Rezeptortyp B, Gen-ID: 5787), PPP1R15A (regulatorische Untereinheit 15A der Proteinphosphatase 1, Gen-ID: 23645), PTPRH (Protein-Tyrosinphosphatase, Rezeptortyp H, Gen-ID: 5794), DDIT3 (durch DNA-Schäden induzierbares Transkript 3, Gen-ID: 1649), BIRC3 (Baculovirales IAP-Wiederholelement mit 3 Einheiten, Gen-ID: 330), F0X03 (Forkhead-Box O3, Gen-ID: 2309), BCL2 (BCL2, Apoptose-Regulator, Gen-ID: 596), PRKCE (Proteinkinase-C epsilon, Gen-ID: 5581), CHMP4C (geladenes multivesikuläres Körperprotein 4C, Gen-ID: 92421), CLDN14 (Claudin 14, Gen-ID: 23562), SLC7A11 (Mitglied 11 der Trägerfamilie für gelöste Stoffe 7, Gen-ID: 23657), BOC (onkogen regulierter, zelladhäsionsassoziierter BOC, Gen-ID: 91653), AJUBA (Ajuba LIM-Protein, Gen-ID: 84962), LMO7 (LIM-Domäne 7, Gen-ID: 4008), MMP24 (Matrix-Metallopeptidase 24, Gen-ID: 10893), C3orf58 (divergente Proteinkinase-Domäne 2A, Gen-ID: 205428), KLF10 (Kruppel-ähnlicher Faktor 10, Gen-ID: 7071), CSGALNACT1 (Chondroitinsulfat-N-acetylgalactosaminyltransferase 1, Gen-ID: 55790), CEP120 (centrosomales Protein 120, Gen-ID: 153241), CHAC1 (ChaC-Glutathion-spezifische gamma-Glutamylcyclotransferase 1, Gen-ID: 79094), ASN (Asparaginsynthetase (Glutamin-hydrolysierend), Gen-ID: 440), NFE2L2 (Kernfaktor, Erythroid-2-ähnliches Element 2, Gen-ID: 4780), RIOK3 (RIO-Kinase 3, Gen-ID: 8780), TXNIP (mit Thioredoxin interagierendes Protein, Gen-ID: 10628), MTR (5-Methyltetrahydrofolat-Homocystein-Methyltransferase, Gen-ID: 4548), IFRD1 (mit Interferon in Beziehung stehender Entwicklungsregulator 1, Gen-ID: 3475), ADGRA2 (Adhäsion-G-Protein-gekoppelter Rezeptor A2, Gen-ID: 25960), TBX4 (T-Box 4, Gen-ID: 9496), OVOL2 (Ovo-ähnlicher Zinkfinger 2, Gen-ID: 58495), ACVRL1 (Aktivin-A-Rezeptor-ähnliches Element Typ 1, Gen-ID: 94), ALB (Albumin, Gen-ID: 213), JADE1 (PHD-Finger 1 der Jade-Familie, Gen-ID: 79960), MLLT11 (MLLT11, co-Faktor des Transkriptionsfaktors 7, Gen-ID: 10962), ADORA1 (Adenosin-Al-Rezeptor, Gen-ID: 134), TNFRSF8 (Mitglied 8 der TNF-Rezeptor-Superfamilie, Gen-ID: 943), NLRP12 (NLR-Familie, pyrindomänenhaltig 12, Gen-ID: 91662), CASP14 (Caspase 14, Gen-ID: 23581), LTA (Lymphotoxin alpha, Gen-ID: 4049), SMAGP (Kleinzelladhäsions-Glycoprotein, Gen-ID: 57228), NEO1 (Neogenin-Vorläufer, Gen-ID: 4756), MTSS1 (MTSS1, I-BAR-domänenhaltig, Gen-ID: 9788), TSPAN32 (Tetraspanin 32, Gen-ID: 10077), CLDN8 (Claudin 8, Gen-ID: 9073), MYBPH (myosinbindendes Protein H, Gen-ID: 4608), SGCE (Sarkoglykan epsilon, Gen-ID: 8910), CDH15 (Cadherin 15, Gen-ID: 1013), ARHGAP6 (Rho-GTPase-aktivierendes Protein 6, Gen-ID: 395), ZFP36L2 (ZFP36 Ringfingerprotein-ähnliches Element 2, Gen-ID: 678), EHF (ETShomologer Faktor, Gen-ID: 26298), SLAMF6 (Mitglied 6 der SLAM-Familie, Gen-ID: 114836), HLA-DPB1 (Haupthistokompatibilitätskomplex, Klasse II, DP Beta 1, Gen-ID: 3115), SSC5D (Mitglied der cysteinreichen Scavenger-Rezeptoren mit 5 Domänen, Gen-ID: 284297), CCR4 (C-C-Motiv-Chemokinrezeptor 4, Gen-ID: 1233), CCR7 (C-C-Motiv-Chemokinrezeptor 7, Gen-ID: 1236), KLRG1 (Mitglied 1 der Unterfamilie G der Killerzellen-Lektin-ähnlichen Rezeptoren, Gen-ID: 10219), BLNK (B-Zelllinker, Gen-ID: 29760), IGFBP1 (Insulin-ähnliches Wachstumsfaktor-Bindungsprotein 1, Gen-ID: 3484), GIF (MIF, Makrophagen-Migrationshemmungsfaktor, Gen-ID: 4282) und einer Kombination davon, wobei die Zubereitung eine Sonde oder einen Primer, die/der auf spezifische Weise an die mRNA oder cDNA eines dieser Gene bindet, oder einen Antikörper oder ein Aptamer umfasst, der/das auf spezifische Weise an das Protein bindet, welches ausgehend von einem dieser Gene exprimiert wird.

## Revendications

1. Utilisation d'une composition pour le diagnostic *in vitro* d'une maladie associée *à Helicobacter pylori,* dans laquelle la composition comprend une préparation pour mesurer la quantité d'expression du gène FGF21 (facteur de croissance des fibroblastes 21, Gene ID : 26291 (identifiant génétique)), ladite préparation comprenant une sonde ou une amorce se liant spécifiquement à l'ARNm ou à l'ADNc dudit gène FGF21, ou un anticorps ou un aptamère se liant spécifiquement à la protéine exprimée par ledit gène FGF21.

2. Utilisation selon la revendication 1, dans laquelle la composition comprend en outre une préparation pour mesurer la quantité d'expression des gènes CTH (cystathionine g-lyase, Gene ID : 1491) ou CREBRF (protéine de liaison à un élément sensible à l'AMPc, Gene ID : 153222), et d'une combinaison de ceux-ci, ladite préparation comprenant une sonde ou une amorce se liant spécifiquement à l'ARNm ou à l'ADNc de l'un desdits gènes, ou un anticorps ou un aptamère se liant spécifiquement à la protéine exprimée par l'un desdits gènes.

3. Utilisation selon la revendication 2, dans laquelle la composition comprend en outre une préparation pour mesurer la quantité d'expression d'un gène sélectionné dans le groupe constitué : de gènes DLL4 (delta-like 4, Gene ID : 54567), FGF18 (facteur de croissance des fibroblastes 18, Gene ID : 8817), FOS (proto-oncogène Fos, sous-unité du facteur de transcription AP-1, Gene ID : 2353), PDK1 (kinase-1 dépendante de phosphoinositide, Gene ID : 5170), PTPRN (phosphatase tyrosine-protéine récepteur de type N, Gene ID : 5798), CLIC4 (canal intracellulaire chlorure 4, Gene ID : 25932), PTPRB (phosphatase tyrosine-protéine, récepteur de type B, Gene ID : 5787), PPP1R15A (sous-unité régulatrice 15A de protéine phosphatase 1, Gene ID : 23645), PTPRH (phosphatase tyrosine-protéine, récepteur de type H, Gene ID : 5794), DDIT3 (transcrit 3 inductible par des dommages à l'ADN, Gene ID : 1649), BIRC3 (baculoviral IAP repeat containing 3, Gene ID : 330), FOXO3 (forkhead box 03, Gene ID : 2309), BCL2 (BCL2, régulateur de l'apoptose, Gene ID : 596), PRKCE (protéine kinase C epsilon, Gene ID : 5581), CHMP4C (protéine de corps multivésiculaire chargée 4C, Gene ID : 92421), CLDN14 (claudine 14, Gene ID : 23562), SLC7A11 (membre 11 de famille de transporteurs de solutés 7, Gene ID : 23657), BOC (adhésion cellulaire associée à BOC, régulée par oncogène, Gene ID : 91653), AJUBA (protéine LIM d'ajuba, Gene ID : 84962), LMO7 (domaine LIM 7, Gene ID : 4008), MMP24 (métallopeptidase matricielle 24, Gene ID : 10893), C3orf58 (protéine kinase divergente domaine 2A, Gene ID : 205428), KLF10 (facteur de type Kruppel 10, Gene ID : 7071), CSGALNACT1 (sulfate de chondroïtine N-acétylgalactosaminyltransférase 1, Gene ID : 55790), CEP120 (protéine centrosomale 120, Gene ID : 153241), CHAC1 (gamma-glutamylcyclotransférase 1 spécifique du glutathion ChaC, Gene ID : 79094), ASNS (asparagine synthétase (hydrolysant la glutamine), Gene ID : 440), NFE2L2 (nuclear factor, erythroid 2 like 2, Gene ID : 4780), RIOK3 (RIO kinase 3, Gene ID : 8780), TXNIP (protéine interagissant avec thiorédoxine, Gene ID : 10628), MTR (5-méthyltétrahydrofolate-homocystéine méthyltransférase, Gene ID : 4548), IFRD1 (régulateur du développement 1 lié à l'interféron, Gene ID : 3475), ADGRA2 (récepteur A2 couplé à la protéine G d'adhésion, Gene ID : 25960), TBX4 (T-box 4, Gene ID : 9496), OVOL2 (doigt de zinc 2 de type ovo, Gene ID : 58495), ACVRL1 (récepteur de l'activine A comme le type 1, Gene ID : 94), ALB (albumine, Gene ID : 213), JADE1 (doigt PHD de la famille jade 1, Gene ID : 79960), MLLT11 (MLLT11, cofacteur du facteur de transcription 7, Gene ID : 10962), ADORA1 (récepteur de l'adénosine Al, Gene ID : 134), TNFRSF8 (membre de la superfamille des récepteurs TNF 8, Gene ID : 943), NLRP12 (domaine pyrine de la famille NLR contenant 12, Gene ID : 91662), CASP14 (caspase 14, Gene ID : 23581), LTA (lymphotoxine alpha, Gene ID : 4049), SMAGP (glycoprotéine d'adhésion à petites cellules, Gene ID : 57228), NEO1 (précurseur 1 de néogénine, Gene ID : 4756), MTSS1 (MTSS1, contenant le domaine I-BAR, Gene ID : 9788), TSPAN32 (tétraspanine 32, Gene ID : 10077), CLDN8 (claudine 8, Gene ID : 9073), MYBPH (protéine de liaison à la myosine H, Gene ID : 4608), SGCE (sarcoglycane epsilon, Gene ID : 8910), CDH15 (cadhérine 15, Gene ID : 1013), ARHGAP6 (protéine d'activation de la GTPase Rho 6, Gene ID : 395), ZFP36L2 (protéine de l'annulaire ZFP36 de type 2, Gene ID : 678), EHF (facteur homologue ETS, Gene ID : 26298), SLAMF6 (membre de la famille SLAM 6, Gene ID : 114836), HLA-DPB1 (complexe majeur d'histocompatibilité, classe II, DP bêta 1, Gene ID : 3115), SSC5D (membre de la famille riche en cystéine du récepteur accepteur avec 5 domaines, Gene ID : 284297), CCR4 (récepteur 4 de chimiokines du motif C-C, Gene ID : 1233), CCR7 (récepteur 7 de chimiokines du motif C-C, Gene ID : 1236), KLRG1 (membre 1 de sous-famille G du récepteur de type lectine des cellules tueuses, Gene ID : 10219), BLNK (lieur des cellules B, Gene ID : 29760), IGFBP1 (facteur de croissance analogue à l'insuline = protéine de liaison 1, Gene ID : 3484), GIF (MIF, facteur inhibiteur de la migration des macrophages, Gene ID : 4282), et d'une combinaison de ceux-ci, ladite préparation comprenant une sonde ou une amorce se liant spécifiquement à l'ARNm ou à l'ADNc de l'un desdits gènes, ou un anticorps ou un aptamère se liant spécifiquement à la protéine exprimée par l'un desdits gènes.

4. Utilisation d'un kit pour le diagnostic *in vitro* d'une maladie associée à *Helicobacter pylori,* dans laquelle le kit comprend la composition telle que définie dans l'une quelconque des revendications 1 à 3.

5. Procédé pour fournir des informations nécessaires au diagnostic d'une maladie associée *à Helicobacter pylori* chez un patient infecté par *Helicobacter pylori,* le procédé comprenant l'étape de détermination du niveau d'expression d'un gène dont l'expression est augmentée ou diminuée par infection à *Helicobacter pylori* dans un échantillon du patient infecté par *Helicobacter pylori,* dans lequel l'étape de détermination du niveau d'expression du gène comprend la mesure de la quantité d'expression du gène FGF21 (facteur de croissance des fibroblastes 21, Gene ID : 26291) à l'aide d'une sonde ou d'une amorce se liant spécifiquement à l'ARNm ou à l'ADNc dudit gène FGF21, ou d'un anticorps ou d'un aptamère se liant spécifiquement à la protéine exprimée par ledit gène FGF21.

6. Procédé pour fournir des informations nécessaires au diagnostic d'une maladie associée *à Helicobacter pylori* selon la revendication 5, dans lequel l'étape de détermination du niveau d'expression du gène comprend en outre la mesure de la quantité d'expression de gènes CTH (cystathionine g-lyase, Gene ID : 1491) ou CREBRF (protéine de liaison à un élément sensible à l'AMPc, Gene ID : 153222), et d'une combinaison de ceux-ci, ladite préparation comprenant une sonde ou une amorce se liant spécifiquement à l'ARNm ou à l'ADNc de l'un desdits gènes, ou un anticorps ou un aptamère se liant spécifiquement à la protéine exprimée par l'un desdits gènes.

7. Procédé pour fournir des informations nécessaires au diagnostic d'une maladie associée *à Helicobacter pylori* selon la revendication 6, dans lequel l'étape de détermination du niveau d'expression du gène comprend en outre la mesure de la quantité d'expression d'un gène sélectionné dans le groupe constitué : de gènes DLL4 (delta-like 4, Gene ID : 54567), FGF18 (facteur de croissance des fibroblastes 18, Gene ID : 8817), FOS (proto-oncogène Fos, sous-unité du facteur de transcription AP-1, Gene ID : 2353), PDK1 (kinase-1 dépendante de phosphoinositide, Gene ID : 5170), PTPRN (phosphatase tyrosine-protéine récepteur de type N, Gene ID : 5798), CLIC4 (canal intracellulaire chlorure 4, Gene ID : 25932), PTPRB (phosphatase tyrosine-protéine, récepteur de type B, Gene ID : 5787), PPP1R15A (sous-unité régulatrice 15A de protéine phosphatase 1, Gene ID : 23645), PTPRH (phosphatase tyrosine-protéine, récepteur de type H, Gene ID : 5794), DDIT3 (transcrit 3 inductible par des dommages à l'ADN, Gene ID : 1649), BIRC3 (baculoviral IAP repeat containing 3, Gene ID : 330), FOXO3 (forkhead box 03, Gene ID : 2309), BCL2 (BCL2, régulateur de l'apoptose, Gene ID : 596), PRKCE (protéine kinase C epsilon, Gene ID : 5581), CHMP4C (protéine de corps multivésiculaire chargée 4C, Gene ID : 92421), CLDN14 (claudine 14, Gene ID : 23562), SLC7A11 (membre 11 de famille de transporteurs de solutés 7, Gene ID : 23657), BOC (adhésion cellulaire associée à BOC, régulée par oncogène, Gene ID : 91653), AJUBA (protéine LIM d'ajuba, Gene ID : 84962), LMO7 (domaine LIM 7, Gene ID : 4008), MMP24 (métallopeptidase matricielle 24, Gene ID : 10893), C3orf58 (protéine kinase divergente domaine 2A, Gene ID : 205428), KLF10 (facteur de type Kruppel 10, Gene ID : 7071), CSGALNACT1 (sulfate de chondroïtine N-acétylgalactosaminyltransférase 1, Gene ID : 55790), CEP120 (protéine centrosomale 120, Gene ID : 153241), CHAC1 (gamma-glutamylcyclotransférase 1 spécifique du glutathion ChaC, Gene ID : 79094), ASNS (asparagine synthétase (hydrolysant la glutamine), Gene ID : 440), NFE2L2 (nuclear factor, erythroid 2 like 2, Gene ID : 4780), RIOK3 (RIO kinase 3, Gene ID : 8780), TXNIP (protéine interagissant avec thiorédoxine, Gene ID : 10628), MTR (5-méthyltétrahydrofolate-homocystéine méthyltransférase, Gene ID : 4548), IFRD1 (régulateur du développement 1 lié à l'interféron, Gene ID : 3475), ADGRA2 (récepteur A2 couplé à la protéine G d'adhésion, Gene ID : 25960), TBX4 (T-box 4, Gene ID : 9496), OVOL2 (doigt de zinc 2 de type ovo, Gene ID : 58495), ACVRL1 (récepteur de l'activine A comme le type 1, Gene ID : 94), ALB (albumine, Gene ID : 213), JADE1 (doigt PHD de la famille jade 1, Gene ID : 79960), MLLT11 (MLLT11, cofacteur du facteur de transcription 7, Gene ID : 10962), ADORA1 (récepteur de l'adénosine Al, Gene ID : 134), TNFRSF8 (membre de la superfamille des récepteurs TNF 8, Gene ID : 943), NLRP12 (domaine pyrine de la famille NLR contenant 12, Gene ID : 91662), CASP14 (caspase 14, Gene ID : 23581), LTA (lymphotoxine alpha, Gene ID : 4049), SMAGP (glycoprotéine d'adhésion à petites cellules, Gene ID : 57228), NEO1 (précurseur 1 de néogénine, Gene ID : 4756), MTSS1 (MTSS1, contenant le domaine I-BAR, Gene ID : 9788), TSPAN32 (tétraspanine 32, Gene ID : 10077), CLDN8 (claudine 8, Gene ID : 9073), MYBPH (protéine de liaison à la myosine H, Gene ID : 4608), SGCE (sarcoglycane epsilon, Gene ID : 8910), CDH15 (cadhérine 15, Gene ID : 1013), ARHGAP6 (protéine d'activation de la GTPase Rho 6, Gene ID : 395), ZFP36L2 (protéine de l'annulaire ZFP36 de type 2, Gene ID : 678), EHF (facteur homologue ETS, Gene ID : 26298), SLAMF6 (membre de la famille SLAM 6, Gene ID : 114836), HLA-DPB1 (complexe majeur d'histocompatibilité, classe II, DP bêta 1, Gene ID : 3115), SSC5D (membre de la famille riche en cystéine du récepteur accepteur avec 5 domaines, Gene ID : 284297), CCR4 (récepteur 4 de chimiokines du motif C-C, Gene ID : 1233), CCR7 (récepteur 7 de chimiokines du motif C-C, Gene ID : 1236), KLRG1 (membre 1 de sous-famille G du récepteur de type lectine des cellules tueuses, Gene ID : 10219), BLNK (lieur des cellules B, Gene ID : 29760), IGFBP1 (facteur de croissance analogue à l'insuline = protéine de liaison 1, Gene ID : 3484), GIF (MIF, facteur inhibiteur de la migration des macrophages, Gene ID : 4282), et d'une combinaison de ceux-ci, ladite préparation comprenant une sonde ou une amorce se liant spécifiquement à l'ARNm ou à l'ADNc de l'un desdits gènes, ou un anticorps ou un aptamère se liant spécifiquement à la protéine exprimée par l'un desdits gènes.
